# EUROPÄISCHE PATENTSCHRIFT

(11) **EP 0 497 803 B1**
(45) Veröffentlichungstag und Bekanntmachung des Hinweises auf die Patenterteilung: **22.12.1993**
(21) Anmeldenummer: 90915406.4
(22) Anmeldetag: 23.10.1990
(51) Int. Cl.: A61F 2/44

(54) **WIRBELKÖRPERIMPLANTAT**
VERTEBRAL IMPLANT
IMPLANT VERTEBRAL

(30) Priorität: 23.10.1989 DE 8912648 U
(43) Veröffentlichungstag der Anmeldung: 12.08.1992
(73) Patentinhaber: Gross, Ulrich, Prof. Dr. med., D-12203 Berlin (DE); SCHMITZ, Herman-Josef, Dr. Dr., D-12165 Berlin (DE); KADEN, Bertram, Dr., D-12157 Berlin (DE); FUHRMANN, Gerard, Dr., D-14167 Berlin (DE)
(72) Erfinder: Gross, Ulrich, Prof. Dr. med., D-12203 Berlin (DE); SCHMITZ, Herman-Josef, Dr. Dr., D-12165 Berlin (DE); KADEN, Bertram, Dr., D-12157 Berlin (DE); FUHRMANN, Gerard, Dr., D-14167 Berlin (DE)
(74) Vertreter: Christiansen, Henning, Dipl.-Ing.
(86) Internationale Anmeldenummer: DE9000819
(87) Internationale Veröffentlichungsnummer: WO9105521

(56) Entgegenhaltungen:
- WO-A-90/00037
- CH-A- 672 589
- DE-A- 2 263 842
- DE-A- 2 804 936
- US-A- 4 863 477
- US-A- 4 865 603

## Beschreibung

Die Erfindung betrifft ein Wirbelkörperimplantat der im Oberbegriff des Anspruchs 1 angegebenen Art.

Eine derartige Endoprothese ist beispielsweise aus der DE-OS 28 04 936 und der DE-OS 22 63 842 bekannt.

Nachteilig bei den bekannten derartigen Endoprothesen ist, daß zur Verankerung in den Wirbelknochen entweder eine komplizierte Implantatform und eine entsprechende Knochenausfräsung oder die Verwendung von Knochenzement erforderlich ist.

Die erreichbare Sicherung gegen Verdrehungen und seitliches Auswandern ist in Anbetracht der starken mechanischen Beanspruchung der Wirbelsäule im allgemeinen problematisch.

Aufgabe der Erfindung ist es daher, eine Endoprothese der Zwischenwirbelscheibe anzugeben, die sich vor allem durch guten und dauerhaften Sitz zwischen den Wirbelkörpern auszeichnet, wobei Mikrobewegungen, insbesondere Verdrehungen und seitliches Auswandern verhindert werden.

Diese Aufgabe wird mit den kennzeichnenden Merkmalen des Anspruchs 1 gelöst.

Die Erfindung beruht auf der Erkenntnis, daß ein seitliches Auswandern eines Wirbelkörperimplantats durch eine Reihe von geometrischen Formgebungsmaßnahmen sicher verhindert werden kann, wobei diese Formgebung bei einem runden im wesentlichen scheibenförmigen Implantat in der Weise erfoigt, daß die Bewegung in jeglicher radialen und auch in tangentialer Richtung verhindernde Sperren in Form von im wesentlichen quer zu dieser Bewegungsrichtung gerichten Fläche vorgesehen sind. Die entsprechende Formgebung ist weiterhin in einer Weise vorgesehen, welche eine Eindringen von die Flächen enthaltenden Teilen in die angrenzenden Wirbel ermöglicht, wobei diese deshalb dachkantförmig geformten sind. Dieses Eindringen nimmt mit der Belastung zu, so daß damit die Sperrwirkung vergrößert wird. Die Oberfläche ist weiterhin so beschaffen, daß sie an die entsprechend durch Anschleifen bearbeiteten Wirbelkörper anwächst. Die Stabilisierung gegen ein unerwünschtes seitliches Verschieben oder Verdrehen des Wirbelkörperimplantats durch geometrische Gestaltung muß also mindestens wirksam sein, bis ein Anwachsen des Implantats erfolgt ist.

Erfindungsgemäß sind die an die Wirbelknochen angrenzenden Stirnflächen der Endoprothese kreisförmig ausgebildet und jeweils mit einer zentrischen Aufbauchung und mehreren dachartigen Vorsprüngen versehen, wobei die Längskanten der Vorsprünge konzentrische Kreisbögen bilden. Die Aufbauchung gestattet eine Zentrierung der Endoprothese relativ zum Wirbelkörper und trägt außerdem zur Lagestabilisierung in bezug auf seitliche Verschiebungen bei. Hauptsächlich jedoch werden Verschiebnungen wie auch Rotationsbewegungen durch die Vorsprünge verhindert.

Gemäß einer vorteilhaften Ausführung der Erfindung sind Größe und Form der Aufbauchung den anatomischen Verhältnissen des Wirbelknochens in der Weise angepaßt, daß sich die Aufbauchung in das weichere Gewebe im Inneren des Wirbelkörpers fest eindrücken läßt. Die Aufbauchung ist dabei vorzugsweise sphärisch konvex ausgebildet.

Die dachartigen Vorsprünge sind in die Spongiosa des Wirbelkörpers eindrückbar, so daß lediglich ein ebener Anschliff des Wirbelkörpers erforderlich ist. Die Firstkanten der dachartigen Vorsprünge sind wie die Grundkanten bevorzugt als konzentrische Kreisbögen ausgebildet, wobei ihre Längen kleiner als die der Grundkanten sind. Dadurch und durch die zentrierte Lage der Firstkanten relativ zu den Grundflächen der Vorsprüngen sind die Giebelflächen als schräg aufragende Dreiecke ausgebildet, wodurch zum einen das Eindrücken des Implantates in den Knochen und zum anderen das Anwachsen an den Knochen erleichtert wird.

Die Anordnung der Vorsprünge ist bevorzugt derart symmetrisch getroffen, daß die Stirnfläche aus mehreren gleichartigen Kreissegmenten zusammengesetzt erscheint. Dabei stimmen die gegenseitigen Abstände, die Abstände von den Rändern der Segmente und die radialen Grundflächenausdehnungen der Vorsprünge überein. Die Längskantenausdehnungen der Vorsprünge nehmen mit radialem Abstand proportional zu. Es hat sich gezeigt, daß bei jeweils vier in radialer Richtung einander benachbart angeordneten Vorsprüngen pro Segment und zwölf Segmenten eine ausreichende Verankerungssicherheit und gute Anwachsbedingungen gegeben sind.

Insbesondere ein starr ausgebildeter Abstandskörper, der den Wirbelbewegungen nicht nachgeben kann und dessen Grenzflächen zu den Wirbelknochen daher besonderen Belastungen ausgesetzt sind, läßt sich auf diese Weise sicher verankern.

Die an die eben angeschliffenen Knochenoberflächen anzupressenden und entsprechend der vorstehenden Beschreibung ausgeformten Stirnflächen der Endoprothese sind vorzugsweise mit einer porösen Beschichtung aus biokompatiblem Material versehen. Eine derartige Schicht besteht bevorzugt aus einer sogenannten Madreporierung aus einem mit dem Kernmaterial identischen Werkstoff oder einer resorbierbaren Beschichtung aus Polylactid oder einem entsprechenden geeigneten Material, welches - auf eine poröse Oberfläche aufgebracht - nach der Implantation durch neu gebildetes Knochenmaterial ersetzt wird.

Eine auf der Mantelfläche des Abstandskörpers umlaufende Rille dient der Vereinfachung des Operationsablaufes. Das Implantat läßt sich bequem mit einer chirurgischen Zange halten und einsetzen. Spongiosaschrauben, Knochenzement oder andere Verankerungshilfsmittel sind nicht erforderlich.

Wenn gemäß einer bevorzugten Ausführung der Erfindung die Stirnfläche eine, insbesondere zur Mittelebene symmetrische, Neigung mit einem Winkel, der vorzugsweise zwischen 3 oder 4° liegt, aufweisen, kommt die Form des Implantats der Krümmung der Wirbelsäule im Halsbereich entgegen und ermöglicht insoweit eine optimale Anpassung und Kraftüberleitung zwischen Wirbel und Implantat.

Insbesondere ist eine verrundete Ausnehmung vorgesehen, welche sich vom Rand her um im wesentlichen 10% des Durchmessers zum Zentrum hin erstreckt und insbesondere um eine senkrechte Achse verrundet ist. Diese Ausnehmung bildet einen Durchlaß für den Spinalnerv und ist vorzugsweise in demjenigen Teil der Umfangs angeordnet zu dem die Stirnflächen hin konvergieren.

Zur erleicherten Manipulation des Implantats ist eine Bohrung vorgesehen ist, welche sich vom Rand her in Richtung auf das Zentrum erstreckt und über mindestens einen Teil ihrer Länge mit einem Innengewinde versehen ist. Zum Eingriff in diese Bohrung ist ein Setzwerkzeug vorgesehen, daß einen Stab oder eine Stange mit einem entsprechenden Außengewinde vorgesehen. Weiterhin ist das Werkzeug mit einer verschiebaren Hülse versehen, welche an ihrem dem Außengewinde des Stabs zugewandten Ende mit mindestens einem nasenartigen Vorsprung versehen ist, welcher in eine der Bohrung des Implantats benachbarte Ausnehmung eingreift, die insbesondere Teil einer den Implantatkörper ringförmig umlaufenden Rille ist. Durch einen Anschlag bzw. einen entsprechenden Mitnehmer wird mit dem Einschrauben des Gewindeteils der Stange in die Bohrung der nasenartige Vorsprung in die Ausnehmung gezogen und dort verriegelt.

Vorteilhafte Weiterbildungen der Erfindung sind in den Unteransprüchen gekennzeichnet bzw. werden nachfolgend zusammen mit der Beschreibung der bevorzugten Ausführung der Erfindung anhand der Figuren näher dargestellt. Es zeigen:
Figur 1 ein Ausführungsbeispiel einer erfindungsgemäßen Endoprothese in längsgeschnittener Darstellung,
Figur 1a eine vergrößerte längsgeschnittene Darstellung des Details Ia der Endoprothese gemäß Figur 1,
Figur 1b eine vergrößerte längsgeschnittene Darstellung des Details Ib der Endoprothese gemäß Figur 1,
Figur 2 eine Draufsicht auf die Endoprothese gemäß Figur 1,
Figur 3 eine perspektivische Ansicht der Endoprothese gemäß den Figuren 1 und 2,
Figuren 4a bis 4d eine weitere Ausführungsvariante der erfindungsgemäßen Endoprothese sowie
Figuren 5a und 5b ein entsprechendes Setzwerkzeug.

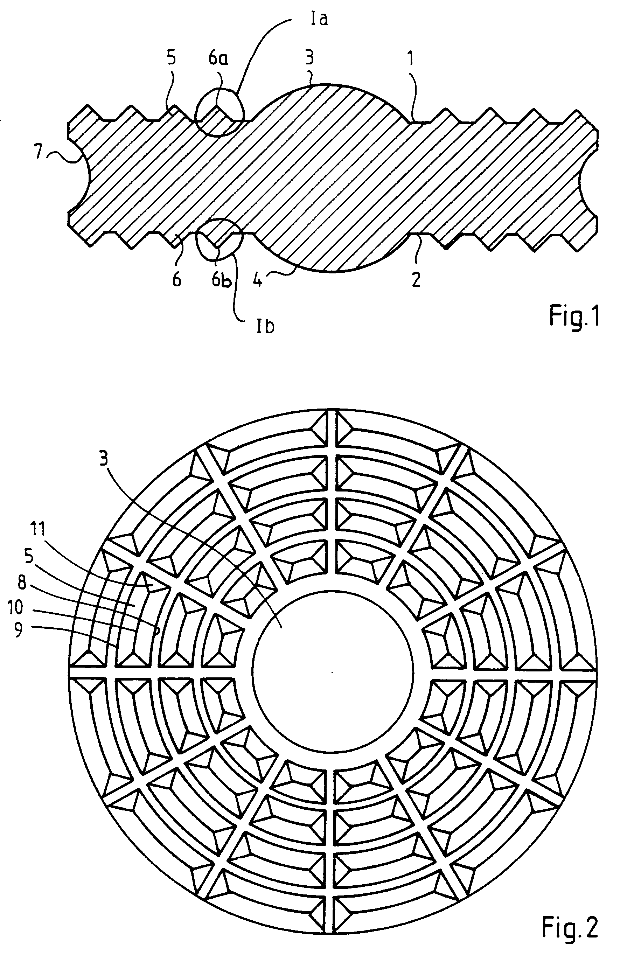

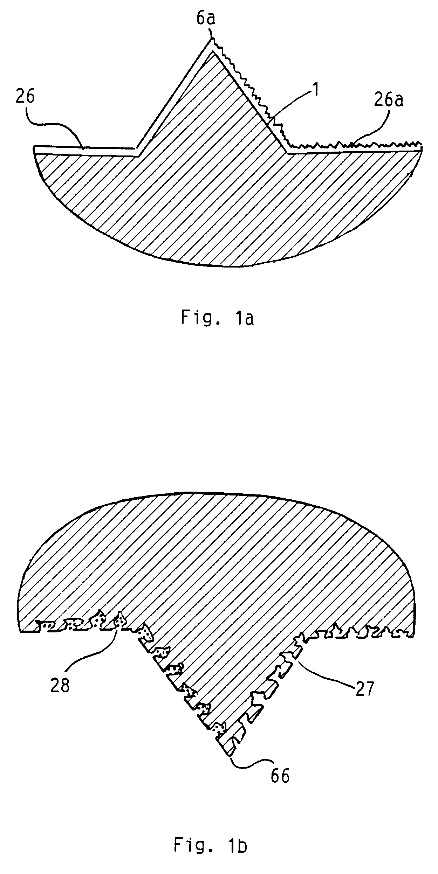

Eine in Figur 1 in einem Längsschnitt wiedergegebene Endoprothese der Zwischenwirbelscheibe weist an beiden, den Wirbelknochen zugewandten Stirnflächen 1 und 2 zentrische, konvex gewölbte Aufbauchungen 3 und 4 auf. Die Aufbauchungen 3 und 4 sind jeweils von dachartigen Vorsprüngen konzentrisch umgeben, von denen zwei als Beispiele mit 5 und 6 bezeichnet sind. Die aufragenden Kanten der Vorsprünge 5 und 5 sind schneidenartig ausgebildet, wodurch das Anpressen der Prothese an die ebenflächig angeschliffenen Wirbelknochen erleichtert wird. Da die Wirbelkörper in ihrem Innern aus weicherem Gewebe bestehen, lassen sich die die Spitzen 6a und 6b der Vorsprünge 5 und 6 noch überragenden zentrischen Aufbauchungen 3 und 4 in das Knochengewebe eindrücken. Zum Ergreifen und Führen mit einer chirurgischen Zange ist die äußere Mantelfläche der Endoprothese mit einer umlaufenden Rille 7 versehen. Diese Rille bildet eine im Querschnitt verrundete Vertiefung, welche sich etwa über die halbe Höhe der Mantelfläche erstreckt.

Figur 1a zeigt eine vergrößerte Darstellung des Details I der Endoprothese der Figur 1. Die Oberfläche der Stirnfläche 1 ist mit einer porösen biokompatiblen Beschichtung 26 versehen. Diese Oberflächenbeschichtung 26 kann entweder eine glatte 26 oder eine rauhe 26a Strukturierung aufweisen. Die behandelte und Poren aufweisende Oberfläche 27 der Stirnfläche 2 in der Fig. 1b ist teilweise mit resorbierbarem Material 28 ausgefüllt um ein Einwachsen von Knochenmaterial zu ermöglichen. Eine solche Oberflächenbehandlung 27 und/oder Oberflächenbeschichtung 26 dient dem verbesserten Anwachsen am Knochen.

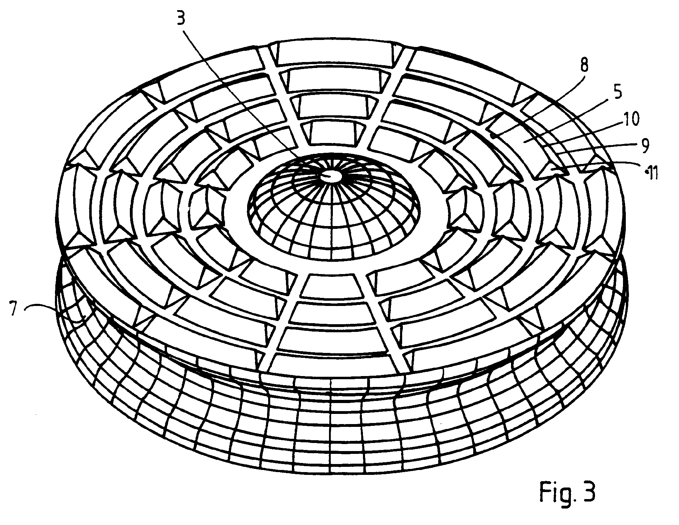

Figur 2 zeigt eine Draufsicht auf eine der beiden gleichartig ausgeformten Stirnflächen 1 oder 2 der Endoprothese. Die kreisförmige Stirnfläche 1 bzw. 2 ist in einen zentrischen Bereich, der von der Aufbauchung 3 bzw. 4 eingenommen wird und vier, die Aufbauchung 3 bzw. 4 ringförmig umgebenden Bereichen gegliedert. Jeder Ringbereich ist mit zwölf dachartigen Vorsprüngen 5 bzw. 6 versehen, wobei die Form und die Größe der Vorsprünge 5 bzw. 6 innerhalb eines Ringbereiches übereinstimmen. Die Oberflächenstruktur der Stirnflächen 1 und 2 weist zwölf gleiche Segmente auf. Die Segmente verhindern zusammen mit der Aufbauchung 3 bzw. 4 ein unerwünschtes Verschieben in jeglicher radialen Richtung.

Die Grundkanten 8 und 9 und die Firstkante 10 der dachartigen Vorsprünge 5 bzw. 6 bilden konzentrische Kreisbögen, wobei die Firstkante 10 eine kleinere Länge als die Grundkanten 8 und 9 aufweist. Dadurch sind die giebelseitigen Begrenzungen 11 der dachartigen Vorsprünge 3 bzw. 4 schräg aufragend ausgebildet. Die Vorsprünge sind damit insgesamt "walmdachartig". Durch diese stirnseitigen Begrenzungen wird auch ein unerwünschtes Verdrehen der Wirbelkörper in beiden tangentialen Richtungen verhindert.

Die kreisbogenförmig ausgebildeten dachkantartigen Vorsprünge 5 bzw. 6 nehmen in ihrer Länge von innen nach außen zu. Sie liegen auf symmetrisch auf Radien und sind strahlenförmig ausgerichtet. Zwischen de Vorsprüngen verbleiben Bereiche, die in der Ebene der Stirnflächen des im wesentlichen zylindrischen Implantats liegen.

Eine perspektivische Ansicht der Endoprothese der Zwischenwirbelsacheibe in der Darstellung als Drahtmodell zur Veranschaulichung der Oberflächengestaltung ist in Figur 3 wiedergegeben.

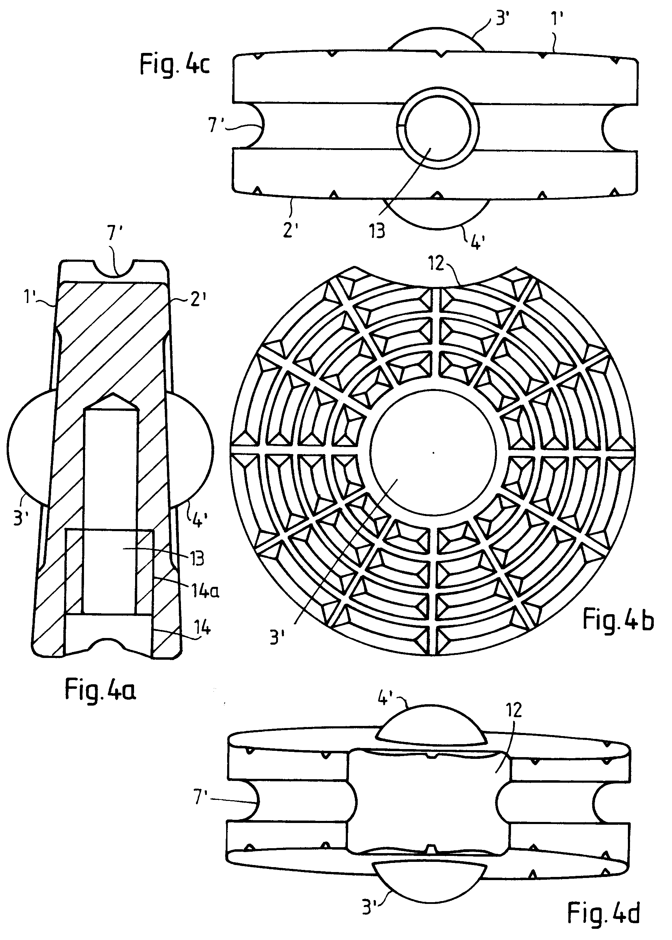

In den Figuren 4a bis d ist eine weitere Variante des erfindungsgemäßen Wirbelkörperimplantats dargestellt. Die mit dem zuvor beschriebenen Ausführungsbeispiel übereinstimmenden Elemente sind mit entsprechenden Bezugszeichen versehen, denen jeweils ein "'" hinzugefügt wurde.

In der Schnittdarstellung von Figur 4a ist zu erkennen, daß die beiden Stirnflächen eine Neigung von ca. 7° relativ zueinander aufweisen. Die Neigungsrichtung ist für bei Stirnflächen symmetrisch zur Mittelebene gewählt und beträgt somit zwischen Mittelebene und Stirnfläche jeweils ca. 3,5°. Der Durchmesser des Implantats beträgt zwischen 16 und 20 mm, während die Höhe ca. 6 bis 9 mm beträgt. Die Gesamtneigung der Stirnflächen wird bei den verschiedenen Wirbelgrößen angepaßten Ausführungen mit zunehmendem Durchmesser vergrößert, so daß die maximale Neigung dem maximalen Durchmesser zuzuordnen ist und umgekehrt. Auf diese Weise ist die Gestaltung der Elemente den anatomischen Gegebenheiten in optimaler Weise angepaßt.

In der Draufsicht gemäß Figur 4b ist eine verrundete Ausnehmung 12 erkennbar, welche eine Tiefe von 10% des Durchmessers aufweist. Sie ist zylindrisch verrundet, wobei sich Achse der Verrundung parallel zur geometrischen Achse des Implantats erstreckt. Die Aussparung verhindert, daß durch das Implantat Nervenbahnen im Wirbelbereich beeinträchtigt werden. Der Radius der Verrundung ist etwa gleich dem halben Radius der Stirnflächen.

Wie auch aus Figur 4d erkennbar ist, befindet sich die Ausnehmung 12 an derjenigen Seite des Implantats, an der die beiden Stirnflächen den geringsten gegenseiten Abstand aufweisen.

Aus der Schnittdarstellung gemäß Figur 4a und auch aus der Seitenansicht gemäß Figur 4c ist erkennbar, daß das hier dargestellte Implantat mit einer Sackbohrung 13 versehen ist, welche sich in etwa um den halben Durchmesser der Erhebung 3' bzw. 4' über den Mittenbereich hinaus erstreckt. Zwischen einem äußeren erweiterten Bereich 14 und dem Ende der Bohrung ist ein metrisches Innengewinde 14a vorgesehen. Der äußere erweiterte Bereich 14 verhindert, daß Knochen in die Gewindebohrung einwächst. Zusätzlich ist hierzu noch eine - in der Zeichnung nicht dargestellte - Verschlußkappe aus einem elastischen bioverträglichen Material, wie beispielsweise Silikonkautschuk, vorgesehen, mit der die Öffnung nach Einbringen des Implantats verschließbar ist.

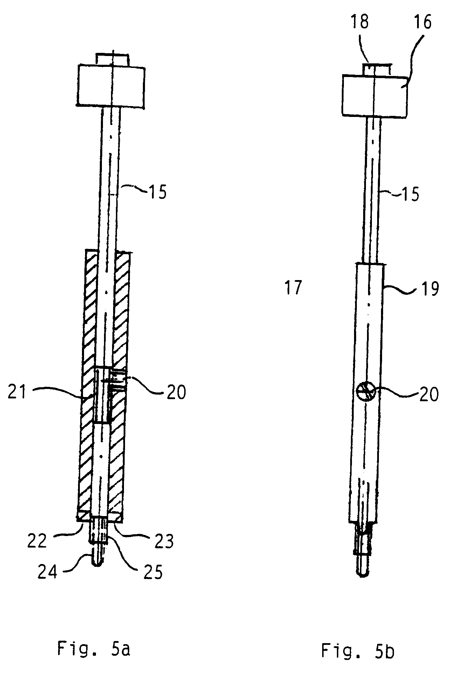

Die Sackbohrung dient zur Aufnahme eines Werkzeugs zum Manipulieren eines Wirbelkörperimplantats, welches in den Figuren 5a in der Draufsicht im Schnitt und in Figur 5b in Seitenansicht dargestellt ist.

Auf einem schlanken Schaftbereich 15 ist ein Schlaggewicht 16 zwischen zwei Anschlägen 17 und 18 verschieblich gelagert, so daß je nach Schlagrichtung ein Ein- bzw. Austreiben des mit dem so gebildeten Setzwerkzeug verbundenen Implantats erfolgen kann.

Der Anschlag 17 wird durch die Stirnseite einer Hülse 19 gebildet, die auf dem Schaft 15 um einen festen Längenbetrag längsverschieblich gelagert ist. Die Hülse 19 ist mit einer Schraube 20 versehen, welche fest mit der Hülse 19 verbunden ist und mit ihrem der Mittelachse der Hülse zugewandten Ende in den Innenraum der Hülse hinein vorsteht. Dieses Ende greift in einen Bereich 21 mit verringertem Durchmesser des Schafts 15 ein. Auf diese Weise ist die Hülse 19 unverlierbar mit dem Schaft 15 verbunden. Die Hülse ist im Querschnitt oval geformt und weist an der dem Anschlag 17 gegenüberliegenden Seite nasenförmige Ansätze 22 und 23 auf, welche in entsprechende Ausnehmungen des Implantats eingreifen können.

Ein freies gewindeloses Schaftende 24 entspricht in seinem Durchmesser dem der Sackbohrung 13 und das daran anschließende Außengewinde 25 ist dem Innengewinde 14a der Bohrung angepaßt.

Zum Einsetzen des Implantats wird das freie mit dem Gewinde 25 versehene Schaftende 24 in die Bohrung des Implantats eingesetzt und dort festgeschraubt. Dabei ziehen sich die Nasen 22 und 23 in die umlaufende Rille des Implants hinein und geben somit die Möglichkeit dessen Ausrichtung durch Ergreifen der Hülse 19 zu kontrollieren. Mit dem Setzgewicht 16 kann jetzt ein Eintreiben des Implantats erfolgen. Nach Erreichen der Endposition wird das Werkzeug entfernt. Zum Entfernen des Implantats bei Reoperationen wird entsprechend in umgekehrter Reihenfolge vorgegangen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Wirbelkörperimplantat bestehend aus einem zwischen benachbarten Wirbelkörpern einsetzbaren Abstandskörper, wobei die an die Wirbelkörper angrenzenden Stirnflächen (1, 2) des Implantats scheibenförmig rund ausgebildet sind,
**dadurch gekennzeichnet**,
daß die angrenzenden Stirnflächen (1, 2) des Implantats jeweils eine zentrale erhabene Aufbauchung (3 bzw. 4) sowie dachkantartige Vorsprünge (5 bzw. 6) aufweisen.

2. Wirbelkörperimplantat nach Anspruch 1, **dadurch** **gekennzeichnet**, daß die Grundkanten (8, 9) und die Firstkanten (10) der Vorsprünge (5 bzw. 6) konzentrische Teile von Kreisbögen bilden.

3. Wirbelkörperimplantat nach Anspruch 1, **dadurch** **gekennzeichnet**, daß die Aufbauchung (3 bzw. 4) sphärisch konvex ausgebildet ist, wobei ihr Außendurchmesser dem Innendurchmesser des Markraums eines Wirbelkörpers angepaßt ist.

4. Wirbelkörperimplantat nach Anspruch 1, **dadurch** **gekennzeichnet**, daß die dachkantartigen Vorsprünge (5, 6) abgeschrägte Stirnflächen (11) aufweisen, in der Weise, daß die Länge der Firstkanten (10) kleiner ist als die der Grundkanten (8, 9) und daß zwischen den dachkantartigen Vorsprüngen (5, 6) ebene Bereiche der Stirnfläche (1, 2) des Wirbelkörpers verbleiben.

5. Wirbelkörperimplantat nach Anspruch 2, **dadurch** **gekennzeichnet**, daß die kreisbogenförmige Firstkanten (10) der dachkantartigen Vorsprünge (5 und 6) in radialer Richtung der Stirnflächen in Richtung nach außen hin zunehmende Längen aufweisen.

6. Wirbelkörperimplantat nach Anspruch 1, **dadurch** **gekennzeichnet**, daß die Oberfläche der Stirnflächen (1, 2) und/oder dachkantartigten Vorsprünge mit einer das Anwachsen am Knochen begünstigenden Be- schichtung (26) bzw. Oberflächengestaltung (27) versehen ist.

7. Wirbelkörperimplantat nach Anspruch 1, **dadurch** **gekennzeichnet**, daß eine auf der Mantelfläche des im wesentlichen zylindrisch ausgebildeten Abstandskörpers ringförmig umlaufende Rille (7) vorgesehen ist.

8. Wirbelkörperimplantat nach Anspruch 1, **dadurch** **gekennzeichnet**, daß der Wirbelkörper drehsymmetrisch und/oder symmetrisch zu seiner Mittelebene ausgebildet ist.

9. Wirbelkörperimplantat nach Anspruch 1, **dadurch** **gekennzeichnet**, daß die Stirnflächen (3' und 4') eine bezogen auf eine Mittelebene symmetrische Neigung zwischen 3 und 4° aufweisen.

10. Wirbelkörperimplantat nach Anspruch 9, **dadurch** **gekennzeichnet**, daß eine insbesondere um eine zur geometrischen Mittelachse parallele geometrische Achse verrundete Ausnehmung (12) vorgesehen ist, welche sich vom Rand her bis zu einer Tiefe von im wesentlichen 10% des Durchmessers zum Zentrum hin erstreckt.

11. Wirbelkörperimplantat nach Anspruch 9 und 10, **dadurch gekennzeichnet**, daß die Ausnehmung (12) in demjenigen Teil der Umfangs angeordnet sind, zu dem die Stirnflächen (3', 4') hin konvergieren.

12. Wirbelkörperimplantat nach Anspruch 1, **dadurch** **gekennzeichnet**, daß eine Bohrung (13) vor- gesehen ist, welche sich vom Rand des Implantats her in Richtung auf das Zentrum erstreckt.

13. Wirbelkörperimplantat nach Anspruch 12, **dadurch gekennzeichnet**, daß die Bohrung über mindestens einen Teil ihrer Länge mit einem Innengewinde (14a) versehen ist.

14. Kombination eines Werkzeugs und eines Implantats nach einem der Ansprüche 12 oder 13, **dadurch** **gekennzeichnet**, daß ein an seinem einen Ende mit einem in das Innengewinde (14a) der Bohrung (14) eingreifenden Außengewinde (25) versehener Schaft (15) vorgesehen ist, der eine verschiebbare Hülse (19) aufweist, welche an ihrem dem Außengewinde des Stabs zugewandten Ende mit mindestens einem nasenartigen Vorsprung (22, 23) versehen ist, der in eine der Bohrung des Implantats benachbarte Ausnehmung eingreift, die insbesondere einen Teil einer den Implantatkörper ringförmig umlaufenden Rille (7') bildet.

15. Kombination eines Werkzeugs und eines Implantats nach Anspruch 14, **dadurch gekennzeichnet**, daß das Werkzeug als Setzwerkzeug ausgebildet und der Schaft dazu ein zwischen zwei Anschlägen verschiebliches Gewicht (16) aufweist.

## Claims

1. Vertebral implant comprising a spacing member to be inserted between adjacent vertebrae, the end faces (1,2) of the implant, adjoining the vertebrae, being circular and disc-shaped in construction, characterised in that the adjacent end faces (1,2) of the implant each have a central raised convexity (3 and 4, respectively) and roof-like projections (5 and 6, respectively).

2. Vertebral implant according to claim 1, characterised in that the base edges (8,9) and the ridge edges (10) of the projections (5 and 6) form concentric parts of arcs of a circle.

3. Vertebral implant according to claim 1,
characterised in that the convexity (3 or 4) is of spherically convex construction, the external diameter thereof being adapted to fit the internal diameter of the medullary cavity of a vertebra.

4. Vertebral implant according to claim 1,
characterised in that the roof-shaped projections (5,6) have chamfered end faces (11) so that the length of the ridge edges (10) is less than that of the base edges (8, 9) and flat areas of the end face (1,2) of the vertebra remain between the roof-like projections (5,6).

5. Vertebral implant according to claim 2,
characterised in that the arcuate ridge edges (10) of the roof-shaped projections (5 and 6) increase in length in the radially outward direction of the end faces.

6. Vertebral implant according to claim 1,
characterised in that the surface of the end faces (1,2) and/or roof-shaped projections is provided with a coating (26) or surface configuration (27) which promotes bone growth thereon.

7. Vertebral implant according to claim 1,
characterised in that an annular encircling groove (7) is provided on the outer surface of the substantially cylindrical spacing member.

8. Vertebral implant according to claim 1,
characterised in that the vertebra is rotationally symmetrical and/or is of symmetrical construction with regard to its central plane.

9. Vertebral implant according to claim 1,
characterised in that the end faces (3' and 4') have an inclination of between 3° and 4° which is symmetrical with respect to a central plane.

10. Vertebral implant according to claim 9,
characterised in that a recess (12) is provided which is rounded particularly about a geometric axis parallel to the geometric central axis, this recess extending from the edge to a depth of substantially 10% of the diameter towards the centre.

11. Vertebral implant according to claims 9 and 10,
characterised in that the recess (12) is provided in that part of the circumference towards which the end faces (3',4') converge.

12. Vertebral implant according to claim 1,
characterised in that a bore (13) is provided which extends from the edge of the implant towards the centre.

13. Vertebral implant according to claim 12,
characterised in that the bore is provided with an internal thread (14a) over at least part of its length.

14. Combination of a tool and an implant according to claim 12 or 13, characterised in that a shaft (15) is provided which has at one end an external thread (25) engaging in the internal thread (14a) of the bore (14) and which has a displaceable sleeve (19), said sleeve being provided at its end closest to the external thread of the rod, with at least one lug-like projection (22,23) which engages in a recess adjacent to the bore of the implant, said recess forming, in particular, a part of an annular groove (7') encircling the implant.

15. Combination of a tool and an implant according to claim 14, characterised in that the tool is constructed as a setting tool and the shaft has for this purpose a weight (16) which is movable between two end stops.

## Revendications

1. Implant vertébral composé d'un corps écarteur insérable entre des corps vertébraux voisins, les faces frontales (1, 2) de l'implant, adjacentes aux corps vertébraux, ayant une forme circulaire de type disque, caractérisé en ce que les faces frontales (1, 2) adjacentes de l'implant présentent chacune un renflement central surélevé (3 ou 4) ainsi que des parties saillantes (5 ou 6) en forme d'arête de toit.

2. Implant vertébral selon la revendication 1, caractérisé en ce que les arêtes basales (8, 9) et les arêtes faîtières (10) des parties saillantes (5 ou 6) forment des parties concentriques d'arcs de cercle.

3. Implant vertébral selon la revendication 1, caractérisé en ce que le renflement (3 ou 4) a une forme sphérique convexe, son diamètre externe étant adapté au diamètre interne du trou rachidien d'un corps vertébral.

4. Implant vertébral selon la revendication 1, caractérisé en ce que les parties saillantes (5, 6) en forme d'arête de toit présentent des faces frontales (11) biseautées, de sorte que les arêtes faîtières (10) sont plus courtes que les arêtes basales (8, 9) et que demeurent entre les parties saillantes (5, 6) en forme d'arête de toit des zones planes de la face frontale (1, 2) du corps vertébral.

5. Implant vertébral selon la revendication 2, caractérisé en ce que les arêtes faîtières (10) en forme d'arcs de cercle des parties saillantes (5 et 6) en forme d'arête de toit présentent des longueurs progressivement croissantes vers l'extérieur, dans la direction radiale des faces frontales.

6. Implant vertébral selon la revendication 1, caractérisé en ce que la surface des faces frontales (1, 2) et/ou des parties saillantes en forme d'arête de toit est dotée d'un revêtement (26) ou d'une configuration superficielle (27) favorisant la soudure avec l'os.

7. Implant vertébral selon la revendication 1, caractérisé en ce qu'est prévue une gorge circonférentielle annulaire sur la face latérale du corps écarteur ayant une forme sensiblement cylindrique.

8. Implant vertébral selon la revendication 1, caractérisé en ce que le corps vertébral est symétrique en rotation et/ou symétrique par rapport à son plan médian.

9. Implant vertébral selon la revendication 1, caractérisé en ce que les faces frontales (3' et 4') présentent une inclinaison, symétrique par rapport à un plan médian, comprise entre 3 et 4°.

10. Implant vertébral selon la revendication 9, caractérisé en ce qu'est prévu un évidement (12) arrondi notamment autour d'un axe géométrique parallèle à l'axe médian géométrique et qui s'étend du bord vers le centre jusqu'à une profondeur sensiblement égale à 10 % du diamètre.

11. Implant vertébral selon les revendications 9 et 10, caractérisé en ce que l'évidement (12) est disposé dans la partie de la circonférence vers laquelle convergent les faces frontales (3', 4').

12. Implant vertébral selon la revendication 1, caractérisé en ce qu'est prévu un trou (13) qui s'étend du bord de l'implant en direction de son centre.

13. Implant vertébral selon la revendication 12, caractérisé en ce que le trou présente, sur au moins une partie de sa longueur, un taraudage (14a).

14. Combinaison d'un outil et d'un implant selon l'une des revendications 12 ou 13, caractérisée en ce qu'est prévue une tige (15) munie à une de ses extrémités d'un filetage mâle (25) s'engrenant dans le taraudage (14a) du trou (14) et qui présente un manchon coulissant (19) doté à son extrémité tournée vers le filetage mâle de la tige d'au moins une partie saillante (22, 23) en forme de téton qui s'engrène dans un évidement, proche du trou de l'implant, qui fait notamment partie d'une gorge annulaire (7') entourant le corps de l'implant.

15. Combinaison d'un outil et d'un implant selon la revendication 14, caractérisée en ce que l'outil a la forme d'un outil de montage et que la tige présente à cet effet un poids (16) coulissant entre deux butées.
